# FASCICULE DE BREVET EUROPEEN

(11) **EP 3 102 249 B1**
(45) Date de publication et mention de la délivrance du brevet: **16.10.2019**
(21) Numéro de dépôt: 15706881.8
(22) Date de dépôt: 03.02.2015
(51) Int. Cl.: A61L 9/01, B22C 1/16, C07C 209/90

(54) **COMPOSITION D'AMINES À ODEUR MASQUÉE**
AMINZUSAMMENSETZUNG MIT ÜBERDECKTEM GERUCH
ODOUR-MASKED AMINE COMPOSITION

(30) Priorité: 06.02.2014 FR 1450914
(43) Date de publication de la demande: 14.12.2016
(73) Titulaire: Arkema France, 92700 Colombes (FR)
(72) Inventeur: RUPPIN, Christophe, 73250 Saint-Pierre d'Albigny (FR); FORQUY, Christian, 64400 Oloron Sainte-Marie (FR)
(86) Numéro de dépôt international: PCT/FR2015/050239
(87) Numéro de publication internationale: WO 2015/118254

(56) Documents cités:
- EP-A1- 0 247 946
- EP-A1- 1 955 792
- EP-A2- 0 141 266
- WO-A1-2005/110499
- WO-A2-2006/138132
- JP-A- H08 302 383

## Description

La présente invention concerne une composition d'amine à odeur masquée, son procédé de préparation, ainsi que les utilisations de ladite composition d'amine à odeur masquée.

Les amines sont des composés organiques aujourd'hui très largement utilisées dans de nombreux secteurs industriels, par exemple en tant que catalyseurs ou agents de réticulation dans le domaine de la synthèse de polymères et en particulier de polyuréthanes, ou encore en tant qu'additifs dans les lubrifiants, dans les peintures, et autres.

Un inconvénient des amines organiques peut dans certains cas provenir de leur odeur, plus ou moins développée, plus ou moins prononcée, et plus ou moins nauséabonde. Certaines amines dégagent même des odeurs tellement fortes et/ou désagréables que leur manipulation doit être réalisée en enceinte close, ou bien encore avec des équipements respiratoires destinés au personnel technique, ce qui n'est pas sans poser des problèmes de logistiques, et des coûts associés.

Ainsi, par exemple, des amines, plus particulièrement des amines tertiaires ou des mélanges d'amines tertiaires, sont aujourd'hui utilisées dans la fabrication de polymères, par exemple de polyuréthanes, pour la fabrication de sièges pour automobiles, pour la fabrication de noyaux pour moules de fonderie, et autres, pour ne citer que quelques domaines d'applications possibles.

Plus spécifiquement, la fabrication de noyaux pour moules de fonderie fait appel à un procédé nommé « procédé Ashland » (ou encore « moulage boîte froide » ou « Cold Box Process » en langue anglaise), procédé dans lequel un liant comprenant une résine de polyol et un isocyanate est ajoutée à une masse de sable à durcir. Un agent de polymérisation est ensuite injecté sous forme d'aérosol dans la masse de sable à durcir contenant le liant, provoquant le durcissement instantané de la résine. Le durcissement est obtenu par réaction de polyaddition entre la résine de polyol et l'isocyanate en présence de l'agent de polymérisation.

Dans ce procédé, l'agent de polymérisation utilisé est constitué d'amine(s) ou d'un mélange d'amines, et plus particulièrement d'amines tertiaires, qui présentent le plus souvent des odeurs fortes et nauséabondes. Lorsque les amines sont injectées et que le durcissement est obtenu, l'excédent d'amines est éliminé par courant d'air de rinçage avant d'être traité. Inévitablement, des traces résiduelles d'amines sont présentes et génèrent de mauvaises odeurs incommodantes qui nuisent autant aux ouvriers des sites industriels.

Pour éliminer les rejets de composés malodorants dans l'atmosphère, des tours de lavage chimique sont généralement utilisées dans les fonderies. Ces tours de lavage font appel à des techniques d'absorption capables de transférer les molécules malodorantes de la phase gazeuse à la phase aqueuse par le biais de colonnes d'échange. Cependant, ce traitement ne permet pas d'éliminer, ou tout au moins réduire efficacement, l'odeur désagréable que génèrent ces amines en amont, c'est-à-dire avant l'utilisation de celles-ci par les ouvriers dans les usines.

La demande de brevet JP8302383 propose une composition parfumante comprenant au moins un aldéhyde, ayant une chaine carbonée en C₆-C₁₅, et au moins un ester. Cette composition permet de masquer l'odeur incommodante des amines tertiaires, et notamment les amines tertiaires utilisées comme agent de polymérisation dans la production de polyuréthane, par pulvérisation de cette composition odorante dans l'air des enceintes polluées par les odeurs desdites amines.

La demande internationale WO2012/121359 propose une composition déodorante comprenant au moins un acide dicarboxylique et au moins un acide tricarboxylique comme agents actifs et des sels d'acides métalliques. Cette composition déodorante est décrite comme permettant d'éliminer les odeurs d'amines. En fait, les acides carboxyliques et les sels d'acides métalliques sont utilisés pour neutraliser les résidus d'amines qui sont ainsi salifiées.

Ainsi, aucune des solutions connues aujourd'hui ne peut remédier aux inconvénients cités *supra.* et il reste un réel besoin d'éliminer, ou au moins diminuer efficacement, les odeurs désagréables que génèrent les amines, et en particulier les amines tertiaires, par un moyen économique et simple.

Les inventeurs ont maintenant découvert qu'il est possible d'odoriser les amines afin d'en masquer, en totalité ou au moins en très grande partie, les odeurs nauséabondes et gênantes pour les utilisateurs et l'environnement.

Ainsi, la présente invention a pour premier objet une composition comprenant :
a) au moins 90% en poids, plus préférentiellement encore au moins 95%, avantageusement au moins 99%, avantageusement encore au moins 99,5% en poids d'au moins une amine primaire, secondaire ou tertiaire, par rapport au poids total de la composition, et
b) au plus 10%, plus préférentiellement encore au plus 5%, avantageusement au plus 1%, avantageusement encore au plus 0,5%, de manière particulièrement préférée au plus 0,25%, de manière encore plus préférée au plus 0,2%, de manière particulièrement préférée au plus 0,1%, de manière encore plus préférée au plus 0,08% en poids d'un agent masquant d'odeur (b), par rapport au poids total de la composition.

Selon un mode de réalisation, ladite au moins une amine présente dans la composition selon la présente invention, est une amine de formule (I): 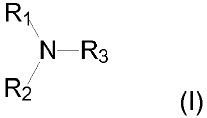 dans laquelle :
- R₁, R₂ et R₃, identiques ou différents, sont choisis indépendamment l'un de l'autre parmi l'atome d'hydrogène, un radical alkyle linéaire ou ramifié comportant de 1 à 12 atomes de carbone, et un radical cyclo-alkyle comprenant de 3 à 12 atomes de carbone,
- deux des substituants choisis parmi R₁, R₂ et R₃ formant éventuellement ensemble et avec l'atome d'azote auxquels ils sont reliés, une structure cyclique comprenant de 2 à 12 atomes de carbone, et éventuellement comprenant un ou plusieurs hétéroatomes choisis parmi oxygène, azote, soufre, phosphore, et éventuellement substitué avec un ou plusieurs groupements fonctionnels choisis parmi hydroxy, alkoxy, alkylcarbonyle, alkoxycarbonyle, benzyle, fluor, chlore, brome, iode, soufre, phosphore et azote.

Selon un mode de réalisation, ledit agent masquant d'odeur comprend :
- au moins un éther de formule (b1) :

   R₄-O-R₅ (b1)

   dans laquelle :
   - R₄ et R₅, identiques ou différents, sont choisis indépendamment l'un de l'autre parmi un radical alkyle linéaire ou ramifié comprenant de 1 à 12 atomes de carbone, un radical cyclo-alkyle comprenant de 3 à 12 atomes de carbone, un radical alcényle comprenant de 2 à 12 atomes de carbone, un radical cyclo-alcényle comprenant de 3 à 12 atomes de carbone, un radical phényle, un radical benzyle,
   - R₄ et R₅ formant éventuellement ensemble et avec l'atome d'oxygène auxquels ils sont reliés, une structure cyclique comprenant de 3 à 20 atomes, et éventuellement comprenant un ou plusieurs hétéroatomes choisis parmi oxygène, azote, soufre, et phosphore, ladite structure cyclique étant éventuellement substituée avec un ou plusieurs groupements choisis parmi hydroxy, alkoxy, alkylcarbonyle, alkoxycarbonyle, phényle, benzyle, fluor, chlore, brome, iode, soufre, phosphore et azote,
- au moins un terpène et/ou un terpénoïde (b2) et
   au moins une oxime de formule (b3) : 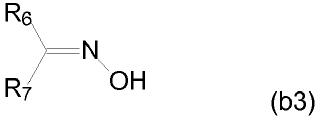 dans laquelle :
   - R₆ est choisi parmi un radical alkyle linéaire ou ramifié comportant de 1 à 24 atomes de carbone, un radical cyclo-alkyle comprenant de 3 à 24 atomes de carbone, un radical alcényle comprenant de 2 à 24 atomes de carbone, un radical cyclo-alcényle comprenant de 3 à 24 atomes de carbone, le radical phényle et le radical benzyle, et R₇ est choisi parmi l'atome d'hydrogène, et un radical alkyle linéaire ou ramifié comportant de 1 à 24 atomes de carbone, un radical cyclo-alkyle comprenant de 3 à 24 atomes de carbone, un radical alcényle comprenant de 2 à 24 atomes de carbone, un radical cyclo-alcényle comprenant de 3 à 24 atomes de carbone, le radical phényle et le radical benzyle,
   - R₆ et R₇ formant éventuellement ensemble et avec l'atome de carbone auxquels ils sont reliés, une structure cyclique comprenant de 3 à 20 atomes, et éventuellement comprenant un ou plusieurs hétéroatomes choisis parmi oxygène, azote, soufre, et phosphore, ladite structure cyclique étant éventuellement substituée avec un ou plusieurs groupements choisis parmi hydroxy, alkoxy, phényle, benzyle, fluor, chlore, brome, iode, soufre, phosphore et azote.

La présente invention a pour objet une composition comprenant :
a) au moins 90% en poids, plus préférentiellement encore au moins 95%, avantageusement au moins 99%, avantageusement encore au moins 99,5% en poids d'au moins une amine primaire, secondaire ou tertiaire, par rapport au poids total de la composition, et
b) au plus 10%, plus préférentiellement encore au plus 5%, avantageusement au plus 1%, avantageusement encore au plus 0,5%, de manière particulièrement préférée au plus 0,25%, de manière encore plus préférée au plus 0,2%, de manière particulièrement préférée au plus 0,1%, de manière encore plus préférée au plus 0,08% en poids d'un agent masquant d'odeur (b), par rapport au poids total de la composition ;
dans laquelle ledit agent masquant d'odeur comprend :
- au moins un éther de formule (b1) :

   R₄-O-R₅ (b1)

   dans laquelle :
   - R₄ et R₅, identiques ou différents, sont choisis indépendamment l'un de l'autre parmi un radical alkyle linéaire ou ramifié comportant de 1 à 12 atomes de carbone, un radical cyclo-alkyle comprenant de 3 à 12 atomes de carbone, un radical alcényle comprenant de 2 à 12 atomes de carbone, un radical cyclo-alcényle comprenant de 3 à 12 atomes de carbone, un radical phényle, un radical benzyle,
   - R₄ et R₅ formant éventuellement ensemble et avec l'atome d'oxygène auxquels ils sont reliés, une structure cyclique comprenant de 3 à 20 atomes, et éventuellement comprenant un ou plusieurs hétéroatomes choisis parmi oxygène, azote, soufre, et phosphore, ladite structure cyclique étant éventuellement substituée avec un ou plusieurs groupements choisis parmi hydroxy, alkoxy, alkylcarbonyle, alkoxycarbonyle, phényle, benzyle, fluor, chlore, brome, iode, soufre, phosphore et azote,
- au moins un terpène et/ou un terpénoïde (b2) et
- au moins une oxime de formule (b3) : 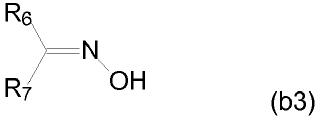 dans laquelle :
   - R₆ est choisi parmi un radical alkyle linéaire ou ramifié comportant de 1 à 24 atomes de carbone, un radical cyclo-alkyle comprenant de 3 à 24 atomes de carbone, un radical alcényle comprenant de 2 à 24 atomes de carbone, un radical cyclo-alcényle comprenant de 3 à 24 atomes de carbone, le radical phényle et le radical benzyle, et R₇ est choisi parmi l'atome d'hydrogène, et un radical alkyle linéaire ou ramifié comportant de 1 à 24 atomes de carbone, un radical cyclo-alkyle comprenant de 3 à 24 atomes de carbone, un radical alcényle comprenant de 2 à 24 atomes de carbone, un radical cyclo-alcényle comprenant de 3 à 24 atomes de carbone, le radical phényle et le radical benzyle,
   R₆ et R₇ formant éventuellement ensemble et avec l'atome de carbone auxquels ils sont reliés, une structure cyclique comprenant de 3 à 20 atomes, et éventuellement comprenant un ou plusieurs hétéroatomes choisis parmi oxygène, azote, soufre, et phosphore, ladite structure cyclique étant éventuellement substituée avec un ou plusieurs groupements choisis parmi hydroxy, alkoxy, phényle, benzyle, fluor, chlore, brome, iode, soufre, phosphore et azote.

Dans la description de la présente invention, sauf mention spécifique contraire, les radicaux alkyle, cycloalkyle, alcényle et cyclo-alcényle peuvent éventuellement être substitués par un ou plusieurs groupements choisis parmi hydroxy, alkoxy, alkylcarbonyle, alkoxycarbonyle, phényle, benzyle, fluor, chlore, brome, iode, soufre, phosphore et azote, et de préférence choisis parmi hydroxy, alkoxy, phényle, benzyle, fluor, chlore, brome, phosphore et azote.

On préfère en outre les radicaux alkyle et alkoxy comportant de 1 à 6 atomes de carbone, de préférence encore de 1 à 4 atomes de carbone.

Selon un mode de réalisation préféré, au moins un des radicaux R₁, R₂ et R₃, ne représente pas l'atome d'hydrogène, l'invention ne visant pas à odoriser l'ammoniac, mais vise à odoriser les amines primaires, secondaires ou tertiaires.

Selon un autre mode de réalisation préféré de l'invention, au moins deux des radicaux R₁, R₂ et R₃, ne représente pas l'atome d'hydrogène, l'invention visant à odoriser les amines secondaires ou tertiaires.

Selon encore un autre mode de réalisation préféré de l'invention, aucun des radicaux R₁, R₂ et R₃ ne représente l'atome d'hydrogène, l'invention visant à odoriser les amines tertiaires.

Selon un mode de réalisation préféré, l'invention concerne une composition comprenant au moins une amine de formule (I) telle que définie précédemment et un agent masquant d'odeur tel que défini précédemment dans les proportions indiquées ci-dessus.

Ainsi, la présente invention offre un moyen simple, efficace et économique permettant de masquer les odeurs des amines, de préférence les amines primaires, secondaires ou tertiaires, de préférence les amines secondaires ou tertiaires, de préférence encore les amines tertiaires, mais aussi les alkylalcanolamines (référencées sous l'acronyme « AAA »), ainsi que les amines comprenant deux, trois ou 4 atomes d'azote (référencées sous la dénomination de « polyamines »).

Comme exemples d'amines primaires qui peuvent être utilisées dans la présente invention, on peut citer de manière non limitative : la propan-1-amine, la propan-2-amine, la cyclopentanamine, la 2-méthylpropan-2-amine, la phénylméthanamine, le 2-aminopentane, le 3-aminopentane, la 1,2-diméthylpropylamine, l'hexylamine, la 1,3-diméthylbutylamine, la n-heptylamine, la n-octylamine, le 2-amino-octane, la 3,3,5-triméthylcyclohexylamine, l'éthylamine (MEA), l'isopropylamine, la *sec*-butylamine, la 3-éthoxypropylamine, la 3-(2-méthoxy-éthoxy)propylamine, la 3-butoxypropylamine, la 3-(2-éthylhexyloxy)propylamine, la 3-*iso*-propoxypropylamine, et la 3-méthoxypropylamine, ainsi que les mélanges de deux ou plusieurs d'entre elles en toutes proportions.

Comme exemples d'amines secondaires qui peuvent être utilisées dans la présente invention, on peut citer de manière non limitative : la N-méthyléthanamine, la N-éthyléthanamine, la N-méthylpentan-3-amine, la N-3-diméthyl-butan-2-amine, la di-*sec*-butylamine, la diamylamine, l'*iso*-propylbenzylamine, la dihexylamine, la diéthylamine, la di-*iso*-propylamine, la N-*iso*-propylméthylamine, la N-butylméthylamine, la N-*sec*-butylméthylamine, la N-*iso*-butylméthylamine, la N-*tert*-butylméthylamine, la N-méthylpentylamine, la N-hexylméthylamine, la N-méthylcyclohexylamine, la N-heptylméthylamine, la N-octylméthylamine, la N-éthylméthylamine, la N-éthylpropylamine, la N-éthyl-*iso*-propylamine, la N-butyléthylamine, la N-*sec*-butyléthylamine, la N-éthylcyclohexylamine, la N-éthylbenzylamine, ainsi que les mélanges de deux ou plusieurs d'entre elles en toutes proportions.

Comme exemples d'amines tertiaires qui peuvent être utilisées dans la présente invention, on peut citer de manière non limitative : la triméthylamine, la N-méthylaziridine, la diméthyléthylamine (DMEA), la N-méthylazétidine, la N-éthylaziridine, la diéthylméthylamine (DEMA), la diméthyl-*iso*-propylamine (DMIPA), la diméthyl-n-propylamine (DMPA), la N-n-propylaziridine, la *N-iso-*propylaziridine, la N-éthylazétidine, la N-méthylpyrrolidine, le N,N,N',N'-tétra-méthyldiaminométhane, la triéthylamine (TEA), la méthyléthyl-n-propylamine, la méthyléthyl-*iso*-propylamine, la diméthyl-n-butylamine, la diméthyl-*sec*-butylamine, la diméthyl-*iso*-butylamine, la diméthyl-*tert*-butylamine, la N-éthylpyrrolidine, la N-méthylpipéridine, l'hexaméthylene-tétramine, la diméthylpipérazine, le N,N,N',N'-tétraméthyldiamino-éthane, les diméthylpentylamines, les méthyléthylbutylamines, les diéthylamines, les dipropylméthylamines, les N-propylpyrrolidines, la N-éthylpipéridine, les diméthylhexylamines, les méthyléthylpentylamines, les diéthylbutylamines, les dipropyléthylamines, les N-butylpyrrolidines, les N-propylpipéridines, la diéthylpipérazine, les diméthylheptylamines, les méthyléthylhexylamines, les diéthylpentylamines, les tripropylamines, les N-pentylpyrrolidines, les N-butylpipéridines, les diméthyloctylamines, les méthyl-éthylheptylamines, les diéthylhexylamines, les éthylpropylpentylamines, les dipropylbutylamines, les N-pentylpipéridines, l'éthyldi-*iso*-propylamine, ainsi que les mélanges de deux ou plusieurs d'entre elles en toutes proportions.

Comme exemples d'alkylalcanolamines de formule (I) qui peuvent être utilisées dans la présente invention, on peut citer de manière non limitative : l'aminopropyldiéthanolamine, le 2,2'-(heptylamino)biséthanol, l'alaninol, le 2-(éthyl-amino)éthanol, le 2-*iso*-propylaminoéthanol, le 2-butylaminoéthanol, le 2-benzyl-aminoéthanol, le 2-(3-aminopropylamino)éthanol, le 2-octylaminoéthanol, le 2-*sec-*butylaminoéthanol, la N-butyldiéthanolamine, la N-sec-butyldiéthanolamine, le 2-(dibutylamino)éthanol, la N-benzyl-N-méthyléthanolamine, la N-heptyldiéthanol-amine, la N-octyldiéthanolamine, ainsi que les mélanges de deux ou plusieurs d'entre elles en toutes proportions.

Comme exemples de polyamines qui peuvent être utilisées dans la présente invention, on peut citer de manière non limitative : la tétraméthyl-propylènediamine (TMPDA), la diéthylaminopropylamine (DEAPA), la diméthyl-aminopropylaminopropylamine (DMAPAPA), le N-méthyl-1,3-diaminopropane, la N-propyl-1,3-propanediamine, la N-*iso*-propyl-1,3-propanediamine, la N,N,N'-tri-méthyl-1,3-propanediamine, la 1-(3-aminopropyl)-2-pyrrolidine, la 3-morpholinopropylamine, la 1-(3-aminopropyl)pipéridine, la 1-(3-aminopropyl)-2-pipécoline, la N-cyclohexyl-1,3-propanediamine, la 3-(dibutylamino)propylamine, la 2-(3-aminopropylamino)éthanol, la N-(aminopropyl)diéthanolamine (APDEA), la 3-(2-aminoéthyl)aminopropylamine, la bis(3-aminopropyl)amine, la méthyl-bis-(3-aminopropyl)amine, le N-(3-aminopropyl)-1,4-diaminobutane, la pentaméthyldipropylènetriamine, le 1,2-bis(3-aminopropylamino)éthane, la N,N'-bis(3-aminopropyl)-1,3-propanediamine, la 1,4-bis-(3-aminopropyl)pipérazine, ainsi que les mélanges de deux ou plusieurs d'entre elles en toutes proportions.

Les amines tout particulièrement préférées mises en oeuvre dans les compositions de la présente invention sont les amines tertiaires choisies parmi la DMEA, la DMIPA, la DEMA, la DMPA et la TEA, ainsi que les mélanges de deux ou plusieurs d'entre elles en toutes proportions. La DMEA, ainsi que les mélanges de DMEA avec une ou plusieurs autres amines de formule (I) sont tout particulièrement préférées.

Selon la présente invention, il est maintenant possible de masquer l'odeur des amines primaires, secondaires et/ou tertiaires, en particulier des amines de formule (I), ainsi que les mélanges de deux ou plusieurs d'entre elles en toutes proportions, telles qu'elles viennent d'être définies. De préférence, l'agent masquant d'odeur ne doit pas réagir avec l'amine à odoriser et est généralement choisi parmi les agents masquant d'odeur porteurs de fonctions qui ne réagissent chimiquement pas avec les fonctions amines, dans les conditions de stockage notamment. Ainsi, on préfère les agents masquant d'odeur ne possédant pas de composés porteurs de fonctions acides, esters, aldéhydes et autres. De tels agents masquant d'odeur peuvent cependant être utilisés, mais toutefois dans des proportions n'excédant pas 0,5% en poids, borne exclue, de préférence 0,4% en poids et encore plus préférentiellement n'excédant pas 0,2% en poids par rapport au poids total d'agents masquant d'odeur. En outre, on préfère les compositions stables dans le temps, présentant une durée minimale de vie en pot (« shelf life » en langue anglaise) supérieure à 1 mois, de préférence supérieure à 3 mois, préférentiellement supérieure à 6 mois, et encore plus préférentiellement supérieure à 12 mois. Comme il sera indiqué plus loin, l'odeur de la ou des amine(s) est masquée efficacement au moyen d'un agent masquant d'odeur comprenant au moins un éther de formule (b1), au moins un terpène et/ou un terpénoïde (b2) et au moins une oxime (b3).

Comme exemples d'éthers (b1) qui peuvent être utilisées comme composant de l'agent masquant d'odeur, on peut citer, de manière non limitative : le phénoxybenzène, le diphényléther, les méthoxynaphtalènes, le 1-méthoxy-4-méthyl-(4-méthylanisole), le 2-(2,4-diméthylcyclohex-3-ène-1-yl)-5-méthyl-(1-méthylpropyl)-1,3-dioxane, le méthoxyméthane, le méthoxyéthane, l'éthoxyéthane, le 2-éthoxypropane, l'oxacyclopropane, l'oxacyclopentane, l'oxacyclohexane, le 1,4-dioxacyclohexane, diméthoxyméthane, l'éther diisopropylique, le tétrahydrofurane, le 1,4-dioxane, les pyranes, les dihydropyranes, les phénylpyranes, les dihydrophénylpyranes, les pyranes et dihydropyranes substitués par un phényle et un alkyle (par exemple méthyle, éthyle, propyle ou butyle) et autres, ainsi que les mélanges de deux ou plusieurs d'entre eux en toutes proportions.

Comme exemples de terpènes et de terpénoïdes (b2) qui peuvent être utilisées comme composant de l'agent masquant d'odeur, on peut citer, de manière non limitative : les terpinènes, le myrcène, le limonène, le terpinolène, les pinènes, le sabinène, le camphène, l'ocimène, l'eucalyptol, le citral, le menthol, le camphre, la menthone, le terpinéol, l'*iso*-bornéol, le nérol, le citronellal, le citronellol, le linalol, le géraniol, le myrcénol, ainsi que les mélanges de deux ou plusieurs d'entre eux en toutes proportions, et également les essences à base de terpènes et/ou terpénoïdes, notamment celles comprenant ces composants.

L'agent masquant d'odeur (b) peut également comprendre une ou plusieurs oximes (b3), et tout particulièrement une aldoxime ou une cétoxime, de préférence une cétoxime. Parmi les oximes (b3), on peut citer à titre d'exemples non limitatifs les oximes pour lesquelles R₇ représente l'atome d'hydrogène, ou un radical choisi parmi méthyle, éthyle n-propyle, *iso*-propyle, n-butyle, *sec*-butyle, et *tert*-butyle et R₆ est choisi parmi les radicaux alkyle linéaires ou ramifiés comportant de 1 à 12 atomes de carbone, de préférence de 1 à 8 atomes de carbone, de préférence encore parmi méthyle, éthyle n-propyle, *iso*-propyle, n-butyle, *sec*-butyle, *tert*-butyle, n-pentyle, *iso*-pentyle, *tert*-pentyle*, neo*-pentyle, et les radicaux hexyle linéaires ou ramifiés.

Selon un aspect préféré, des exemples non limitatifs d'oximes (b3) comprennent la cinnamaldéhyde-oxime, la 2-méthylbutanal oxime, la 3-méthylbutanal oxime, la méthyléthylcétoxime, la 3-heptanone oxime, la 5-méthyl-3-heptanone oxime, la glyoxime, la diméthylglyoxime, la diaminoglyoxime, la pralidoxime, l'obidoxime, la périllartine, le chlorure d'asoxime et la salicylaldoxime, ainsi que les mélanges de deux ou plusieurs d'entre elles en toutes proportions.

Lorsqu'il est fait référence à des intervalles, les expressions de type « allant de...à » incluent les bornes de l'intervalle. À l'inverse, les expressions de type « compris entre...et... » excluent les bornes de l'intervalle. Lorsqu'il est fait référence à des seuils de valeurs, les expressions « au plus » ou « au moins » excluent respectivement la valeur maximum et la valeur minimum. Sauf mention contraire, les pourcentages sont donnés en valeurs pondérales.

Les quantités respectives de chacun des constituants (b1), (b2) et (b3) de l'agent masquant d'odeur (b) peuvent varier dans de larges proportions, selon la nature des constituants (b1), (b2) et (b3) et selon la nature de l'amine ou des amines dont on veut masquer l'odeur.

Selon un mode de réalisation particulièrement préféré, la composition selon l'invention comprend, en % en poids par rapport au poids total de l'agent masquant d'odeur :
- de 1% à 98,9%, de préférence de 2% à 98,9%, de préférence de 5% à 98,9%, de préférence encore de 10% à 98,9%, d'au moins un éther (b1),
- de 1% à 98,9%, de préférence de 2% à 98,9%, de préférence de 5% à 98,9%, de préférence encore de 10% à 98,9%, d'au moins un terpène et/ou terpénoïde (b2),
- de 0,1% à 10%, de préférence de 0,1% à 5%, de préférence de 0,1% à 2%, de préférence encore de 0,1% à 1%, d'au moins une oxime (b3), et
- éventuellement un ou plusieurs additifs, q.s.p. 100%.

Selon un autre mode de réalisation particulièrement préféré, la composition selon l'invention comprend, en % en poids par rapport au poids total de l'agent masquant d'odeur :
- de 1% à 98%, de préférence de 2% à 98%, de préférence de 5% à 98%, de préférence encore de 10% à 98%, d'au moins un éther (b1),
- de 1% à 98%, de préférence de 2% à 98%, de préférence de 5% à 98%, de préférence encore de 10% à 98%, d'au moins un terpène et/ou terpénoïde (b2),
- de 1% à 10%, de préférence de 1% à 5%, de préférence encore de 1% à 2%, d'au moins une oxime (b3), et
- éventuellement un ou plusieurs additifs, q.s.p. 100%.

Parmi les agents masquant d'odeur (b), on préfère en outre ceux comprenant au moins deux éthers différents, de préférence encore ceux comprenant au moins trois éthers différents.

Les additifs qui peuvent être présent dans les agents masquant (b) peuvent être de tous types connus de l'homme du métier, et avantageusement ceux connus de l'homme du métier et compatibles avec l'application envisagée.

Des exemples non limitatifs d'additifs qui peuvent être utilisés dans les agents masquant d'odeur (b) comprennent les solvants, pigments, colorants, conservateurs, anti-oxydants, arômes, et autres.

Les additifs éventuellement présents dans l'agent masquant d'odeur peuvent comporter une ou plusieurs fonctions chimiques choisies parmi aldéhyde, cétone, ester, et alcool. Selon un mode de réalisation préféré, les additifs sont choisis parmi ceux comportant une ou plusieurs fonctions ester. Les additifs comportant au moins une fonction alcool ne sont pas préférés.

Parmi les additifs de type aldéhyde ou cétone, on peut citer de manière non limitative le géranial, le néral, le citronellal, les damascones, les damascénones, les ionones, les irisones, les méthylionones, la frambinone, la dynascone, la menthone, l'iso-menthone, la flavonone et leurs mélanges.

Parmi les additifs de type ester, on peut citer de manière non limitative les esters d'acides en C₂-C₂₀ saturés ou insaturés, tels que les acétates, propionates, butyrates, méthylbutyrates, pentanoates, hexanoates, heptanoates, caproates, oléates, linoléates, linolénates, d'éthyle, de propyle, de butyle, de pentyle, de 2-méthylbutyle, d'iso-amyle, d'hexyle, de benzyle, de phényléthyle, de menthyle, de carvyle, mais aussi les ortho-phtalates, tel que l'ortho-phtalate de diéthyle, les citrates, tels que le citrate de triéthyle, les malonates, tel que le malonate de diéthyle, et autres, ainsi que les mélanges de deux ou plusieurs d'entre eux en toutes proportions.

Parmi les additifs de type alcool, on peut citer de manière non limitative les mono-alcools comprenant de 1 à 30 atomes de carbone, de préférence de 6 à 20 atomes de carbone, de préférence encore de 8 à 11 atomes de carbone, lesdits atomes de carbone formant une chaîne linéaire ou ramifiée comportant éventuellement une ou plusieurs insaturation(s) sous forme de double(s) liaison(s), et comportant éventuellement une structure cyclique à 5 ou 6 chaînons, saturée, ou totalement ou partiellement insaturée choisis parmi le menthol, le néo-menthol, l'alcool phényléthylique, l'alcool benzylique, le citronellol, le dihydromyrcénol, le dihydrotérpinéol, le linalol, l'éthyllinalol, le tétrahydrolinalol, le tétrahydromyrcénol, le géraniol, le nérol, et autres, ainsi que les mélanges de deux ou plusieurs d'entre eux en toutes proportions.

L'agent masquant d'odeur (b) ainsi défini offre l'avantage de masquer de manière particulièrement efficace l'odeur désagréable des amines, et particulièrement des amines tertiaires, sans modifier la structure chimique de celles-ci. En outre, et comme indiqué précédemment,l'agent masquant d'odeur ne doit pas réagir avec l'amine à odoriser et doit également stable au cours du temps dans la composition dans laquelle il est incorporé selon l'invention.

La présente invention a également pour objet un procédé de préparation de ladite composition d'amines à odeur masquée comprenant une étape de mélange d'au moins une amine, de préférence une amine de formule (I) avec au moins un agent masquant d'odeur (b).

Le mélange peut être réalisé selon toute méthode connue de l'homme du métier, et notamment par introduction d'au moins un agent masquant d'odeur (b), éventuellement mais avantageusement sous forme liquide, à température ambiante ou en chauffant, avec ou sans agitation, dans au moins une amine de formule (I), par tous moyens connus de l'homme du métier, tel que par exemple, et de manière non limitative, par pompe doseuse, canne plongeante dans un bac de stockage, pulvérisation, et autres.

La préparation de la composition selon l'invention peut par exemple être effectuée sous pression atmosphérique, à une température comprise entre 0°C et 100°C, de préférence comprise entre la température ambiante et environ 80°C. La préparation peut également être effectuée sous pression ou sous dépression, à des températures comprises dans les gammes indiquées ci-dessus.

La composition selon l'invention peut être sous forme liquide ou gazeuse et dans une gamme de concentration prédéterminée comme vu *supra,* seule ou en combinaison avec un gaz inerte. Le gaz inerte peut-être l'azote, l'air ou le dioxyde de carbone. De préférence, la composition selon l'invention est sous forme liquide à température et pression ambiantes.

Selon un mode de réalisation, la composition selon la présente invention comprenant au moins une amine de formule (I) et au moins un agent masquant d'odeur (b), tels que définis précédemment, peut être diluée par un ou plusieurs solvants avant emploi. Les solvants de dilution qui peuvent être utilisés peuvent être de tous types connus de l'homme du métier, et comprennent par exemple les solvants et mélanges de solvants aqueux, organiques ou hydro-organiques. On préfère en outre les solvants qui sont compatibles avec l'application envisagée. Dans un mode de réalisation particulièrement préféré, le solvant est choisi parmi l'eau, l'éthanol, le n-propanol, l'*iso*-propanol, le n-butanol, l'*iso*-butanol, et les mélanges de deux ou plusieurs d'entre eux en toutes proportions. Selon un aspect tout particulièrement préféré, la composition selon l'invention est diluée à l'eau, et dans un mode de réalisation particulièrement avantageux l'eau de dilution est l'eau présente dans la ou les amines de formule (I), par exemple l'eau présente ou formée lors de la synthèse de la ou des amines de formule (I).

Il a été découvert de manière surprenante que l'ajout d'au moins un agent masquant d'odeur (b) dans au moins une amine de formule (I) permet de réduire de façon tout à fait satisfaisante, voire d'éliminer, les odeurs gênantes ou nauséabondes de ladite au moins une amine.

Ainsi, et selon un autre objet, la présente invention concerne l'utilisation d'au moins un agent masquant d'odeur (b) comprenant au moins un éther, au moins un terpène et/ou un terpénoïde et au moins une oxime, pour masquer l'odeur d'au moins une amine, de préférence au moins une amine de formule (I), de préférence au moins une amine secondaire ou tertiaire de formule (I), de préférence encore au moins une amine tertiaire de formule (I), formule (I) telle que définie précédemment.

Selon un mode de réalisation préféré de cet objet, la présente invention concerne l'utilisation d'au moins un agent masquant d'odeur (b) tel que défini précédemment, pour masquer l'odeur d'un mélange d'au moins deux amines, de préférence d'au moins deux amines tertiaires, de formule (I), telle que défini précédemment.

La composition comprenant au moins une amine de formule (I) et au moins un masquant d'odeur (b) tels qu'ils viennent d'être définis selon l'invention peut être utilisée pour de nombreuses applications et de manière générale peut être utilisée comme toutes composition d'amine(s) ne comportant pas d'agent masquant d'odeur. En effet, et comme autre avantage, la composition de la présente invention est stable, et la ou les amines comprises dans cette composition ne subissent pas de dégradation, telle que pertes de propriétés, jaunissement et autres.

La composition selon la présente invention peut être ainsi utilisée comme toute autre amine ou mélange d'amine ne comprenant d'agent masquant d'odeur, la composition de l'invention présentant une odeur améliorée par rapport au(x) même(s) amine(s) ne comprenant pas d'agent masquant d'odeur (b).

Comme exemples non limitatifs d'applications possibles pour la composition selon la présente invention, on peut citer celles dans lesquelles les amines sont utilisées comme intermédiaires de synthèse en chimie fine, et en particulier de produits pharmaceutiques et de produits pour l'agrochimie, comme capteurs d'acide lors de synthèses de produits chimiques, comme catalyseurs et agents de réticulation pour la préparation de polymères, comme additifs pour lubrifiants et pour peintures, et autres.

Comme exemple tout particulièrement préféré d'utilisation de la composition selon la présente invention, on peut citer la fabrication de polymères, par exemple de polyuréthanes, notamment pour la fabrication de sièges pour automobiles, ou tout particulièrement pour la fabrication de moules de fonderie, notamment de noyaux pour moules de fonderie, selon par exemple la méthode « Ashland » décrite dans le document EP-A-1 955 792.

En effet, lors de l'utilisation de la composition selon l'invention dans le domaine de la fonderie, il a été découvert que ladite composition utilisée comme agent de polymérisation était compatible avec les composés et les conditions mises en oeuvre dans ledit procédé « Ashland » ou procédé dit « Cold Box Process ». L'agent masquant d'odeur permet de masquer de manière particulièrement efficace l'odeur désagréable des amines utilisées, et ceci, sans réagir avec les différents composés nécessaires à la formation d'un moule de fonderie.

En particulier, la composition selon la présente invention peut être utilisée dans les mêmes conditions réactionnelles que les amines, et notamment les amines tertiaires mises en oeuvre comme décrit dans le document EP-A-1 955 792, c'est-à-dire sous forme vaporisée, et typiquement sous forme vaporisée en mélange avec un gaz inerte.

Ainsi, et selon encore un autre objet, la présente invention concerne un procédé de fabrication de moules de fonderie comprenant au moins les étapes suivantes :
a) préparation d'un mélange comprenant un liant et un agrégat, ledit agrégat étant de préférence du sable,
b) mise en forme dans un moule du mélange obtenu à l'étape a),
c) mise en contact du mélange mis en forme avec au moins une composition selon la présente invention, sous forme liquide ou gazeuse, de préférence sous forme gazeuse, et éventuellement avec un vecteur inerte,
d) réticulation du mélange liant/agrégat en un moule de fonderie de forme dure, solide, et réticulée, et
e) récupération du moule de fonderie.

Le terme « moule de fonderie » désigne aussi les noyaux de fonderie, autrement dit les pièces du moule permettant de réaliser les évidements intérieurs d'une pièce moulée ou de zones de celle-ci en contre-dépouille.

Le vecteur inerte éventuellement utilisé à l'étape c) est avantageusement un gaz inerte ou un mélange de gaz inertes, par exemple l'azote et/ou le dioxyde de carbone.

Dans le procédé de préparation d'un moule de fonderie décrit ci-dessus, la composition de l'invention comprend au moins une amine, de préférence tertiaire, de manière tout particulièrement préférée un mélange d'au moins deux amines dont une au moins, voire deux au moins sont des amines tertiaires, au moins un agent masquant (b) et éventuellement de l'eau, et lorsque l'eau est présente, de préférence sa teneur n'excède pas 1%, de préférence encore n'excède pas 0,5% en poids, par rapport au poids total (eau, amine(s) et agent(s) masquant d'odeur).

Selon encore un autre aspect, la présente invention concerne un moule de fonderie substantiellement obtenu selon le procédé décrit ci-dessus, et concerne plus particulièrement un moule de fonderie obtenu par réticulation d'un liant mélangé à un agrégat au moyen d'une composition selon l'invention comprenant au moins une amine de formule (I) telle que défini précédemment et au moins un agent masquant d'odeur (b) tel que défini précédemment.

Les exemples suivants illustrent l'invention sans la limiter.

### EXEMPLES

### Exemple 1: Composition à base d'amine tertiaire à odeur masquée

Afin de caractériser une composition parfumante pouvant servir d'agent masquant, i.e. permettant d'éliminer, de diminuer, d'améliorer, l'odeur d'une amine tertiaire, une procédure de test olfactif et un examen de stabilité ont été mis au point.

### Conditions Opératoires :

Pour réaliser ce test olfactif, une quantité de 1 mL de composition (amine et agent masquant d'odeur) est placée dans un récipient. Ensuite, le récipient contenant la composition est placé dans un dessiccateur de 10 Litres.

Le dessiccateur est laissé à l'air libre et à température ambiante (25°C). Après évaporation totale (environ 30 minutes), un panel composé de 10 personnes inhalent la composition afin de noter l'odeur (test hédonique).

Lorsque les panélistes ont senti les compositions, ils référencent l'odeur de la composition. Ils attribuent, selon leur préférence, une ou plusieurs croix à chacune des compositions à tester. Le nombre de croix données par les panélistes va de 1 (produit le plus désagréable) à 3 (produit le plus agréable).

Pour l'examen de stabilité, les compositions sont stockées à température ambiante (25°C) pendant une durée allant de 1 mois à plus de 1 an.

### Préparation des échantillons de test :

Chaque composition est préparée avec une amine tertiaire, la diméthyléthylamine (DMEA) fournie par la société ARKEMA. La DMEA a une pureté supérieure ou égale à 99%.

Un échantillon de référence est réalisé avec la DMEA pure, il est nommé A1. Trois échantillons comprenant 99,92% en poids par rapport au poids total de l'échantillon de DMEA et 0,08% en poids par rapport au poids total de l'échantillon d'une composition parfumante sont également préparés. Les échantillons sont nommés : A₂, A₃ et A₄.

La nature de l'agent masquant de chacun des échantillons A₂, A₃ et A₄ est donnée ci-dessous, où les pourcentages sont exprimés en poids par rapport au poids total de l'agent masquant :
Échantillon A₂ (test comparatif) : Agent Masquant d'Odeur : AMO-A2
   - 30% à 70% d'esters (acétate de 2-*tert*-butylcyclohexyle, butanoate de 3-méthylbutyle, 2-propylène-3-cyclohexylpropanoate, 4-undécanolide, acétate d'*iso*-pentyle),
   - 15% à 35 % de cétones (5-méthyl-3-heptanone, 1-cyclooct-3-ényléthanone),
   - 15% à 35% d'éthers (1,3,4,6,7,8-hexahydro-4,6,6,7,8,8-hexaméthylindéno[5,6-c]-pyrane),
   où la somme des composants représente 100% en poids de l'agent masquant.
Échantillon A₃ (test comparatif) : Agent Masquant d'Odeur : AMO-A3
   - 30% à 50% d'esters (acétate de 3a,4,5,6,7,7a-hexahydro-4,7-méthano-1-indén-5(6)-yle, benzoate de méthyle, 2-acétyl-1,2,3,4,5,6,7,8-octahydro-2,3,8,8-tétra-méthyl-naphtalène),
   - 30% à 50% de cétones et aldéhydes (3-méthyl-4-(2,6,6-triméthyl-2-cyclohexén-1-yl)-3-butèn-2-one, 2-méthylundécanal, heptan-2-one, 1-(2,6,6-triméthylcyclohex-2-en-1-yl)pent-1-en-3-one, 3-phényl-2-propénal, 3-phénylbutanal)
   - 1% à 10% d'alcools (3,7-diméthyloctan-3-ol, 3,7-diméthyl-2,6-octadién-1-ol),
   - 1% à 10% d'éthers (2-méthoxynaphtalène),
   où la somme des composants représente 100% en poids de l'agent masquant.
Échantillon A₄ (selon l'invention) : Agent Masquant d'Odeur : AMO-B1
   - 10% à 98% d'éthers (phénoxybenzène, 2-méthoxynaphtalène),
   - de 10% à 98% de terpènes/terpénoïdes (limonène, eucalyptol),
   - de 1% à 10% d'oximes (5-méthyl-3-heptanone oxime)
   - de 0,1% à 1% d'additifs, principalement des esters (citrate de triéthyle, tétradécanoate d'isopropyle),
   où la somme des composants représente 100% en poids de l'agent masquant.

### Résultats :

Les résultats de l'exemple 1 sont reproduits dans le Tableau 1 suivant :

**-- Tableau 1 --**

| **Échantillon à tester** | **Odeur** | **Stabilité au stockage** (à température ambiante) |
|---|---|---|
| A₁ | + | Incolore, Stable (plus d'un an) |
| A₂ | ++ | Jaune (après 1 mois), Instable |
| A₃ | ++ | Jaune (après 1 mois), Instable |
| A₄ | ++++ | Incolore, Stable (après 3 mois) |

Dans l'exemple 1 de la présente invention, la perception de l'odeur de la composition selon l'invention A₄ est nettement plus agréable que dans les échantillons A₁, A₂ et A₃.

Par ailleurs, l'échantillon A₄ reste incolore et stable après 3 mois de stockage à température ambiante alors que les échantillons A₂ et A₃, instables, se colorent en jaune après 1 mois de stockage à température ambiante.

### Exemple 2: Composition à base d'amine tertiaire à odeur masquée selon l'invention

On reproduit le mode opératoire de l'exemple 1 pour préparer deux nouveaux échantillons A₅ et A₆, qui diffèrent de l'échantillon A₄ par la teneur en agent masquant, comme indiqué ci-dessous :
Échantillon A₄ (selon l'invention) :
   - DMEA : 99,92% en poids par rapport au poids total de l'échantillon, et
   - agent masquant d'odeur AMO-B1 : 0,08% en poids par rapport au poids total de l'échantillon.
Échantillon A₅ (selon l'invention) :
   - DMEA : 99,86% en poids par rapport au poids total de l'échantillon, et
   - agent masquant d'odeur AMO-B1 : 0,14% en poids par rapport au poids total de l'échantillon.
Échantillon A₆ (selon l'invention) :
   - DMEA : 99,75% en poids par rapport au poids total de l'échantillon, et
   - agent masquant d'odeur AMO-B1 : 0,25% en poids par rapport au poids total de l'échantillon.

### Résultats :

Les résultats de l'exemple 2 sont reproduits dans le Tableau 2 suivant :

**-- Tableau 2 --**

| **Échantillon à tester** | **Odeur** | **Stabilité au stockage** (à température ambiante) |
|---|---|---|
| A₁ | + | Incolore, Stable (plus d'un an) |
| A₄ | ++++ | Incolore, Stable (après 3 mois) |
| A₅ | ++++ | Incolore, Stable (après 3 mois) |
| A₆ | ++ | Incolore, Stable (après 3 mois) |

Les résultats du Tableau 2 montrent que la perception de l'odeur des compositions selon l'invention A₄, A₅ et A₆ est nettement plus agréable que dans l'échantillon A₁, et que l'augmentation de la dose d'agent masquant d'odeur conduit à une légère diminution de la perception olfactive agréable de la composition.

Par ailleurs, les échantillons A₄, A₅ et A₆ restent incolore et stables après 3 mois de stockage à température ambiante, quelle que soit la quantité d'agent masquant incorporée dans le DMEA.

## Revendications

1. Composition comprenant :
a) au moins 90% en poids, plus préférentiellement encore au moins 95%, avantageusement au moins 99%, avantageusement encore au moins 99,5% en poids d'au moins une amine primaire, secondaire ou tertiaire, par rapport au poids total de la composition, et
b) au plus 10%, plus préférentiellement encore au plus 5%, avantageusement au plus 1%, avantageusement encore au plus 0,5%, de manière particulièrement préférée au plus 0,25%, de manière encore plus préférée au plus 0,2%, de manière particulièrement préférée au plus 0,1%, de manière encore plus préférée au plus 0,08% en poids d'un agent masquant d'odeur (b), par rapport au poids total de la composition ;
dans laquelle ledit agent masquant d'odeur comprend :
- au moins un éther de formule (b1) :
R₄-O-R₅ (b1)
dans laquelle :
• R₄ et R₅, identiques ou différents, sont choisis indépendamment l'un de l'autre parmi un radical alkyle linéaire ou ramifié comportant de 1 à 12 atomes de carbone, un radical cyclo-alkyle comprenant de 3 à 12 atomes de carbone, un radical alcényle comprenant de 2 à 12 atomes de carbone, un radical cyclo-alcényle comprenant de 3 à 12 atomes de carbone, où les radicaux alkyle, cycloalkyle, alcényle et cyclo-alcényle peuvent éventuellement être substitués par un ou plusieurs groupements choisis parmi hydroxy, alkoxy, alkylcarbonyle, alkoxycarbonyle, phényle, benzyle, fluor, chlore, brome, iode, soufre, phosphore et azote, et de préférence choisis parmi hydroxy, alkoxy, phényle, benzyle, fluor, chlore, brome, phosphore et azote, un radical phényle, un radical benzyle,
• R₄ et R₅ formant éventuellement ensemble et avec l'atome d'oxygène auxquels ils sont reliés, une structure cyclique comprenant de 3 à 20 atomes, et éventuellement comprenant un ou plusieurs hétéroatomes choisis parmi oxygène, azote, soufre, et phosphore, ladite structure cyclique étant éventuellement substituée avec un ou plusieurs groupements choisis parmi hydroxy, alkoxy, alkylcarbonyle, alkoxycarbonyle, phényle, benzyle, fluor, chlore, brome, iode, soufre, phosphore et azote,
- au moins un terpène et/ou un terpénoïde (b2) et
- au moins une oxime de formule (b3) : 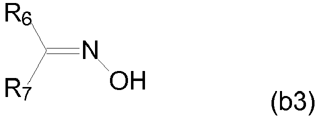 dans laquelle :
• R₆ est choisi parmi un radical alkyle linéaire ou ramifié comportant de 1 à 24 atomes de carbone, un radical cyclo-alkyle comprenant de 3 à 24 atomes de carbone, un radical alcényle comprenant de 2 à 24 atomes de carbone, un radical cyclo-alcényle comprenant de 3 à 24 atomes de carbone, le radical phényle et le radical benzyle, et R₇ est choisi parmi l'atome d'hydrogène, et un radical alkyle linéaire ou ramifié comportant de 1 à 24 atomes de carbone, un radical cyclo-alkyle comprenant de 3 à 24 atomes de carbone, un radical alcényle comprenant de 2 à 24 atomes de carbone, un radical cyclo-alcényle comprenant de 3 à 24 atomes de carbone, le radical phényle et le radical benzyle, où les radicaux alkyle, cycloalkyle, alcényle et cyclo-alcényle peuvent éventuellement être substitués par un ou plusieurs groupements choisis parmi hydroxy, alkoxy, alkylcarbonyle, alkoxycarbonyle, phényle, benzyle, fluor, chlore, brome, iode, soufre, phosphore et azote, et de préférence choisis parmi hydroxy, alkoxy, phényle, benzyle, fluor, chlore, brome, phosphore et azote, R₆ et R₇ formant éventuellement ensemble et avec l'atome de carbone auxquels ils sont reliés, une structure cyclique comprenant de 3 à 20 atomes, et éventuellement comprenant un ou plusieurs hétéroatomes choisis parmi oxygène, azote, soufre, et phosphore, ladite structure cyclique étant éventuellement substituée avec un ou plusieurs groupements choisis parmi hydroxy, alkoxy, phényle, benzyle, fluor, chlore, brome, iode, soufre, phosphore et azote.

2. Composition selon la revendication 1, dans laquelle ladite au moins une amine est une amine de formule (I) : 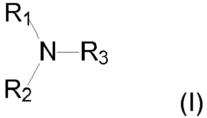 dans laquelle :
- R₁, R₂ et R₃, identiques ou différents, sont choisis indépendamment l'un de l'autre parmi l'atome d'hydrogène, un radical alkyle linéaire ou ramifié comportant de 1 à 12 atomes de carbone, et un radical cyclo-alkyle comprenant de 3 à 12 atomes de carbone, où les radicaux alkyle et cyclo-alkyle peuvent éventuellement être substitués par un ou plusieurs groupements choisis parmi hydroxy, alkoxy, alkylcarbonyle, alkoxycarbonyle, phényle, benzyle, fluor, chlore, brome, iode, soufre, phosphore et azote, et de préférence choisis parmi hydroxy, alkoxy, phényle, benzyle, fluor, chlore, brome, phosphore et azote,
- deux des substituants choisis parmi R₁, R₂ et R₃ formant éventuellement ensemble et avec l'atome d'azote auxquels ils sont reliés, une structure cyclique comprenant de 2 à 12 atomes de carbone, et éventuellement comprenant un ou plusieurs hétéroatomes choisis parmi oxygène, azote, soufre, phosphore, et éventuellement substitué avec un ou plusieurs groupements fonctionnels choisis parmi hydroxy, alkoxy, alkylcarbonyle, alkoxycarbonyle, benzyle, fluor, chlore, brome, iode, soufre, phosphore et azote.

3. Composition selon la revendication 1 comprenant au moins une amine de formule (I) selon la revendication 2.

4. Composition selon la revendication 1, dans laquelle ladite au moins une amine est choisie parmi la propan-1-amine, la propan-2-amine, la cyclopentanamine, la 2-méthylpropan-2-amine, la phénylméthanamine, le 2-aminopentane, le 3-aminopentane, la 1,2-diméthylpropylamine, l'hexylamine, la 1,3-diméthylbutylamine, la n-heptylamine, la n-octylamine, le 2-amino-octane, la 3,3,5-triméthylcyclohexylamine, l'éthylamine (MEA), l'isopropylamine, la *sec-*butylamine, la 3-éthoxypropylamine, la 3-(2-méthoxyéthoxy)propylamine, la 3-butoxypropylamine, la 3-(2-éthylhexyloxy)propylamine, la 3-*iso*-propoxypropylamine, et la 3-méthoxypropylamine, ainsi que les mélanges de deux ou plusieurs d'entre elles en toutes proportions.

5. Composition selon la revendication 1, dans laquelle ladite au moins une amine est choisie parmi la N-méthyléthanamine, la N-éthyléthanamine, la N-méthylpentan-3-amine, la N-3-diméthylbutan-2-amine, la di-*sec*-butylamine, la diamylamine, l'*iso*-propylbenzylamine, la dihexylamine, la diéthylamine, la di-*iso-*propylamine, la N-*iso*-propylméthylamine, la N-butylméthylamine, la N-*sec-*butylméthylamine, la N-*iso*-butylméthylamine, la N-*tert*-butylméthylamine, la N-méthylpentylamine, la N-hexylméthylamine, la N-méthylcyclohexylamine, la N-heptylméthylamine, la N-octylméthylamine, la N-éthylméthylamine, la N-éthylpropylamine, la N-éthyl-*iso*-propylamine, la N-butyléthylamine, la N-*sec*-butyléthylamine, la N-éthylcyclohexylamine, la N-éthylbenzylamine, ainsi que les mélanges de deux ou plusieurs d'entre elles en toutes proportions.

6. Composition selon la revendication 1, dans laquelle ladite au moins une amine est choisie parmi la triméthylamine, la N-méthylaziridine, la diméthyléthylamine (DMEA), la N-méthylazétidine, la N-éthylaziridine, la diéthylméthylamine (DEMA), la diméthyl-*iso*-propylamine (DMIPA), la diméthyl-n-propylamine (DMPA), la N-n-propylaziridine, la N-*iso*-propylaziridine, la N-éthylazétidine, la N-méthylpyrrolidine, le N,N,N',N'-tétraméthyldiaminométhane, la triéthylamine (TEA), la méthyléthyl-n-propylamine, la méthyléthyl-*iso-*propylamine, la diméthyl-n-butylamine, la diméthyl-sec-butylamine, la diméthyl-*iso-*butylamine, la diméthyl-*tert*-butylamine, la N-éthylpyrrolidine, la N-méthylpipéridine, l'hexaméthylene-tétramine, la diméthylpipérazine, le N,N,N',N'-tétra-méthyldiamino-éthane, les diméthylpentylamines, les méthyléthylbutylamines, les diéthylamines, les diproprylméthylamines, les N-propylpyrrolidines, la N-éthylpipéridine, les diméthylhexylamines, les méthyléthylpentylamines, les diéthylbutylamines, les dipropyléthylamines, les N-butylpyrrolidines, les N-propylpipéridines, la diéthylpipérazine, les diméthylheptylamines, les méthyléthylhexylamines, les diéthylpentylamines, les tripropylamines, les N-pentylpyrrolidines, les N-butylpipéridines, les diméthyloctylamines, les méthyl-éthylheptylamines, les diéthylhexylamines, les éthylpropylpentylamines, les dipropylbutylamines, les N-pentylpipéridines, l'éthyldi-*iso*-propylamine, ainsi que les mélanges de deux ou plusieurs d'entre elles en toutes proportions.

7. Composition selon la revendication 1, dans laquelle l'amine est choisie parmi la DMEA, la DMIPA, la DEMA, la DMPA et la TEA, ainsi que les mélanges de deux ou plusieurs d'entre elles en toutes proportions.

8. Composition selon l'une quelconque des revendications précédentes, dans laquelle l'amine est la DMEA, ainsi que les mélanges de DMEA avec une ou plusieurs autres amines de formule (I).

9. Composition selon l'une quelconque des revendications précédentes, dans laquelle l'agent masquant d'odeur comprend au moins un éther (b1) choisi parmi le phénoxybenzène, le diphényléther, les méthoxynaphtalènes, le 1-méthoxy-4-méthyl-(4-méthylanisole), le 2-(2,4-diméthylcyclohex-3-ène-1-yl)-5-méthyl-(1-méthylpropyl)-1,3-dioxane, le méthoxyméthane, le méthoxyéthane, l'éthoxyéthane, le 2-éthoxypropane, l'oxacyclopropane, l'oxacyclopentane, l'oxacyclohexane, le 1,4-dioxacyclohexane, diméthoxyméthane, l'éther di-iso-propylique, le tétrahydrofurane, le 1,4-dioxane, les pyranes, les dihydropyranes, les phénylpyranes, les dihydrophénylpyranes, les pyranes et dihydropyranes substitués par un phényle et un alkyle et autres, ainsi que les mélanges de deux ou plusieurs d'entre eux en toutes proportions.

10. Composition selon l'une quelconque des revendications précédentes, dans laquelle l'agent masquant d'odeur comprend au moins un terpène/terpénoïde (b2) choisi parmi les terpinènes, le myrcène, le limonène, le terpinolène, les pinènes, le sabinène, le camphène, l'ocimène, l'eucalyptol, le citral, le menthol, le camphre, la menthone, le terpinéol, l'*iso*-bornéol, le nérol, le citronellal, le citronellol, le linalol, le géraniol, le myrcénol, ainsi que les mélanges de deux ou plusieurs d'entre eux en toutes proportions, et également les essences à base de terpènes et/ou terpénoïdes, notamment celles comprenant ces composants.

11. Composition selon l'une quelconque des revendications précédentes, dans laquelle l'agent masquant d'odeur comprend au moins une oxime (b3) choisie parmi la cinnamaldéhyde-oxime, la 2-méthylbutanal oxime, la 3-méthylbutanal oxime, la méthyléthylcétoxime, la 3-heptanone oxime, la 5-méthyl-3-heptanone oxime, la glyoxime, la diméthylglyoxime, la diaminoglyoxime, la pralidoxime, l'obidoxime, la périllartine, le chlorure d'asoxime et la salicylaldoxime, ainsi que les mélanges de deux ou plusieurs d'entre elles en toutes proportions.

12. Utilisation d'au moins un agent masquant d'odeur (b) comprenant au moins un éther, au moins un terpène et/ou un terpénoïde et au moins une oxime, pour masquer l'odeur d'au moins une amine, de préférence au moins une amine de formule (I), de préférence au moins une amine secondaire ou tertiaire de formule (I), de préférence encore au moins une amine tertiaire de formule (I) selon la revendication 2.

13. Utilisation d'au moins une composition selon l'une des revendications 1 à 11, pour la fabrication de polymères, ou de moules de fonderie, en particulier de noyaux pour moules de fonderie.

14. Moule de fonderie obtenu par réticulation d'un liant mélangé à un agrégat au moyen d'une composition selon l'une quelconque des revendications 1 à 11.

## Patentansprüche

1. Zusammensetzung, umfassend:
a) mindestens 90 Gew.-%, noch weiter bevorzugt mindestens 95 Gew.-%, vorteilhafterweise mindestens 99 Gew.-%, noch vorteilhafter mindestens 99,5 Gew.-%, mindestens eines primären, sekundären oder tertiären Amins, bezogen auf das Gesamtgewicht der Zusammensetzung, und
b) höchstens 10 Gew.-%, noch weiter bevorzugt höchstens 5 Gew.-%, vorteilhafterweise höchstens 1 Gew.-%, noch vorteilhafter höchstens 0,5 Gew.-%, besonders bevorzugt höchstens 0,25 Gew.-%, noch weiter bevorzugt höchstens 0,2 Gew.-%, besonders bevorzugt höchstens 0,1 Gew.-%, noch weiter bevorzugt höchstens 0,08 Gew.-%, eines Geruchsmaskierungsmittels (b), bezogen auf das Gesamtgewicht der Zusammensetzung;
wobei das Geruchsmaskierungsmittel Folgendes umfasst:
- mindestens einen Ether der Formel (b1):
**R₄-O-R₅** **(b1)**
wobei:
• R₄ und R₅ gleich oder verschieden sind und unabhängig voneinander aus einem linearen oder verzweigten Alkylrest mit 1 bis 12 Kohlenstoffatomen, einem Cycloalkylrest mit 3 bis 12 Kohlenstoffatomen, einem Alkenylrest mit 2 bis 12 Kohlenstoffatomen, einem Cycloalkenylrest mit 3 bis 12 Kohlenstoffatomen, wobei die Alkyl-, Cycloalkyl-, Alkenyl- und Cycloalkenylreste gegebenenfalls durch eine oder mehrere Gruppen, die aus Hydroxy, Alkoxy, Alkylcarbonyl, Alkoxycarbonyl, Phenyl, Benzyl, Fluor, Chlor, Brom, Iod, Schwefel, Phosphor und Stickstoff und vorzugsweise aus Hydroxy, Alkoxy, Phenyl, Benzyl, Fluor, Chlor, Brom, Phosphor und Stickstoff ausgewählt sind, substituiert sein können, einem Phenylrest und einem Benzylrest ausgewählt sind,
• R₄ und R₅ gegebenenfalls zusammen mit dem Sauerstoffatom, an das sie gebunden sind, eine Ringstruktur bilden, die 3 bis 20 Atome umfasst und gegebenenfalls ein oder mehrere Heteroatome, die aus Sauerstoff, Stickstoff, Schwefel und Phosphor ausgewählt sind, umfasst, wobei die Ringstruktur gegebenenfalls durch eine oder mehrere Gruppen, die aus Hydroxy, Alkoxy, Alkylcarbonyl, Alkoxycarbonyl, Phenyl, Benzyl, Fluor, Chlor, Brom, Iod, Schwefel, Phosphor und Stickstoff ausgewählt sind, substituiert sein kann,
- mindestens ein Terpen und/oder ein Terpenoid (b2) und
- mindestens ein Oxim der Formel (b3): 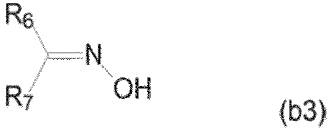 wobei:
• R₆ aus einem linearen oder verzweigten Alkylrest mit 1 bis 24 Kohlenstoffatomen, einem Cycloalkylrest mit 3 bis 24 Kohlenstoffatomen, einem Alkenylrest mit 2 bis 24 Kohlenstoffatomen, einem Cycloalkenylrest mit 3 bis 24 Kohlenstoffatomen, dem Phenylrest und dem Benzylrest ausgewählt ist und R₇ aus einem Wasserstoffatom und einem linearen oder verzweigten Alkylrest mit 1 bis 24 Kohlenstoffatomen, einem Cycloalkylrest mit 3 bis 24 Kohlenstoffatomen, einem Alkenylrest mit 2 bis 24 Kohlenstoffatomen, einem Cycloalkenylrest mit 3 bis 24 Kohlenstoffatomen, dem Phenylrest und dem Benzylrest ausgewählt ist, wobei die Alkyl-, Cycloalkyl-, Alkenyl- und Cycloalkenylreste gegebenenfalls durch eine oder mehrere Gruppen, die aus Hydroxy, Alkoxy, Alkylcarbonyl, Alkoxycarbonyl, Phenyl, Benzyl, Fluor, Chlor, Brom, Iod, Schwefel, Phosphor und Stickstoff und vorzugsweise aus Hydroxy, Alkoxy, Phenyl, Benzyl, Fluor, Chlor, Brom, Phosphor und Stickstoff ausgewählt sind, substituiert sein können,
• R₆ und R₇ gegebenenfalls zusammen mit dem Kohlenstoffatom, an das sie gebunden sind, eine Ringstruktur bilden, die 3 bis 20 Atome umfasst und gegebenenfalls ein oder mehrere Heteroatome, die aus Sauerstoff, Stickstoff, Schwefel und Phosphor ausgewählt sind, umfasst, wobei die Ringstruktur gegebenenfalls durch eine oder mehrere Gruppen, die aus Hydroxy, Alkoxy, Phenyl, Benzyl, Fluor, Chlor, Brom, Iod, Schwefel, Phosphor und Stickstoff ausgewählt sind, substituiert sein kann.

2. Zusammensetzung nach Anspruch 1, wobei es sich bei dem mindestens einen Amin um ein Amin der Formel (I) handelt: 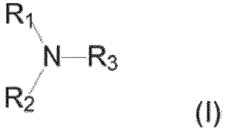 wobei:
- R₁, R₂ und R₃ gleich oder verschieden sind und unabhängig voneinander aus einem Wasserstoffatom, einem linearen oder verzweigten Alkylrest mit 1 bis 12 Kohlenstoffatomen und einem Cycloalkylrest mit 3 bis 12 Kohlenstoffatomen ausgewählt sind, wobei die Alkyl- und Cycloalkylreste gegebenenfalls durch eine oder mehrere Gruppen, die aus Hydroxy, Alkoxy, Alkylcarbonyl, Alkoxycarbonyl, Phenyl, Benzyl, Fluor, Chlor, Brom, Iod, Schwefel, Phosphor und Stickstoff und vorzugsweise aus Hydroxy, Alkoxy, Phenyl, Benzyl, Fluor, Chlor, Brom, Phosphor und Stickstoff ausgewählt sind, substituiert sein können,
- zwei der Substituenten, die aus R₁, R₂ und R₃ ausgewählt sind, gegebenenfalls zusammen mit dem Stickstoffatom, an das sie gebunden sind, eine Ringstruktur bilden, die 2 bis 12 Kohlenstoffatome umfasst und gegebenenfalls ein oder mehrere Heteroatome, die aus Sauerstoff, Stickstoff, Schwefel und Phosphor ausgewählt sind, umfasst und gegebenenfalls durch eine oder mehrere funktionelle Gruppen, die aus Hydroxy, Alkoxy, Alkylcarbonyl, Alkoxycarbonyl, Benzyl, Fluor, Chlor, Brom, Iod, Schwefel, Phosphor und Stickstoff ausgewählt sind, substituiert ist.

3. Zusammensetzung nach Anspruch 1, umfassend mindestens ein Amin der Formel (I) nach Anspruch 2.

4. Zusammensetzung nach Anspruch 1, wobei das mindestens eine Amin aus Propan-1-amin, Propan-2-amin, Cyclopentanamin, 2-Methylpropan-2-amin, Phenylmethanamin, 2-Aminopentan, 3-Aminopentan, 1,2-Dimethylpropylamin, Hexylamin, 1,3-Dimethylbutylamin, n-Heptylamin, n-Octylamin, 2-Aminooctan, 3, 3, 5-Trimethylcyclohexylamin, Ethylamin (MEA), Isopropylamin, *sec*-Butylamin, 3-Ethoxypropylamin, 3-(2-Methoxyethoxy)propylamin, 3-Butoxypropylamin, 3-(2-Ethylhexyloxy)propylamin, 3-Isopropoxypropylamin und 3-Methoxypropylamin sowie Mischungen von zwei oder mehr davon in beliebigen Anteilen ausgewählt ist.

5. Zusammensetzung nach Anspruch 1, wobei das mindestens eine Amin aus N-Methylethanamin, N-Ethylethanamin, N-Methylpentan-3-amin, N-3-Dimethylbutan-2-amin, Di(*sec*-butyl)amin, Diamylamin, Isopropylbenzylamin, Dihexylamin, Diethylamin, Diisopropylamin, N-Isopropylmethylamin, N-Butylmethylamin, N-(*sec*-Butyl)methylamin, N-Isobutylmethylamin, N-(*tert*-Butyl)methylamin, N-Methylpentylamin, N-Hexylmethylamin, N-Methylcyclohexylamin, N-Heptylmethylamin, N-Octylmethylamin, N-Ethylmethylamin, N-Ethylpropylamin, N-Ethylisopropylamin, N-Butylethylamin, N-(sec-Butyl)ethylamin, N-Ethylcyclohexylamin und N-Ethylbenzylamin sowie Mischungen von zwei oder mehr davon in beliebigen Anteilen ausgewählt ist.

6. Zusammensetzung nach Anspruch 1, wobei das mindestens eine Amin aus Trimethylamin, N-Methylaziridin, Dimethylethylamin (DMEA), N-Methylazetidin, N-Ethylaziridin, Diethylmethylamin (DEMA), Dimethylisopropylamin (DMIPA), Dimethyl(n-propyl)amin (DMPA), N-(n-Propyl)aziridin, N-Isopropylaziridin, N-Ethylazetidin, N-Methylpyrrolidin, N,N,N',N'-Tetramethyldiaminomethan, Triethylamin (TEA), Methylethyl(n-propyl)amin, Methylethylisopropylamin, Dimethyl(n-butyl)amin, Dimethyl(*sec*-butyl)amin, Dimethylisobutylamin, Dimethyl(*tert*-butyl)amin, N-Ethylpyrrolidin, N-Methylpiperidin, Hexamethylentetramin, Dimethylpiperazin, N,N,N',N'-Tetramethyldiaminoethan, Dimethylpentylaminen, Methylethylbutylaminen, Diethylaminen, Dipropylmethylaminen, N-Propylpyrrolidinen, N-Ethylpiperidin, Dimethylhexylaminen, Methylethylpentylaminen, Diethylbutylaminen, Dipropylethylaminen, N-Butylpyrrolidinen, N-Propylpiperidinen, Diethylpiperazin, Dimethylheptylaminen, Methylethylhexylaminen, Diethylpentylaminen, Tripropylaminen, N-Pentylpyrrolidinen, N-Butylpiperidinen, Dimethyloctylaminen, Methylethylheptylaminen, Diethylhexylaminen, Ethylpropylpentylaminen, Dipropylbutylaminen, N-Pentylpiperidinen und Ethyldiisopropylamin sowie Mischungen von zwei oder mehr davon in beliebigen Anteilen ausgewählt ist.

7. Zusammensetzung nach Anspruch 1, wobei das Amin aus DMEA, DMIPA, DEMA, DMPA und TEA sowie Mischungen von zwei oder mehr davon in beliebigen Anteilen ausgewählt ist.

8. Zusammensetzung nach einem der vorhergehenden Ansprüche, wobei es sich bei dem Amin um DMEA sowie Mischungen von DMEA mit einem oder mehreren anderen Aminen der Formel (I) handelt.

9. Zusammensetzung nach einem der vorhergehenden Ansprüche, wobei das Geruchsmaskierungsmittel mindestens einen Ether (b1) umfasst, der aus Phenoxybenzol, Diphenylether, Methoxynaphthalinen, 1-Methoxy-4-methyl-(4-methylanisol), 2-(2,4-Dimethylcyclohex-3-en-1-yl)-5-methyl-(1-methylpropyl)-1,3-dioxan, Methoxymethan, Methoxyethan, Ethoxyethan, 2-Ethoxypropan, Oxacyclopropan, Oxacyclopentan, Oxacyclohexan, 1,4-Dioxacyclohexan, Dimethoxymethan, Diisopropylether, Tetrahydrofuran, 1,4-Dioxan, Pyranen, Dihydropyranen, Phenylpyranen, Dihydrophenylpyranen und Pyranen und Dihydropyranen, die durch ein Phenyl und ein Alkyl und andere substituiert sind, sowie Mischungen von zwei oder mehr davon in beliebigen Anteilen ausgewählt ist.

10. Zusammensetzung nach einem der vorhergehenden Ansprüche, wobei das Geruchsmaskierungsmittel mindestens ein Terpen/Terpenoid (b2) umfasst, das aus Terpinenen, Myrcen, Limonen, Terpinolen, Pinenen, Sabinen, Camphen, Ocimen, Eucalyptol, Citral, Menthol, Campher, Menthon, Terpineol, Isoborneol, Nerol, Citronellal, Citronellol, Linalool, Geraniol und Myrcenol sowie Mischungen von zwei oder mehr davon in beliebigen Anteilen und außerdem Essenzen auf der Basis von Terpenen und/oder Terpenoiden, insbesondere denjenigen, die diese Bestandteile umfassen, ausgewählt ist.

11. Zusammensetzung nach einem der vorhergehenden Ansprüche, wobei das Geruchsmaskierungsmittel mindestens ein Oxim (b3) umfasst, das aus Zimtaldehydoxim, 2-Methylbutanaloxim, 3-Methylbutanaloxim, Methylethylketoxim, 3-Heptanonoxim, 5-Methyl-3-heptanonoxim, Glyoxim, Dimethylglyoxim, Diaminoglyoxim, Pralidoxim, Obidoxim, Perillartin, Asoximchlorid und Salicylaldoxim sowie Mischungen von zwei oder mehr davon in beliebigen Anteilen ausgewählt ist.

12. Verwendung mindestens eines Geruchsmaskierungsmittels (b), das mindestens einen Ether, mindestens ein Terpen und/oder ein Terpenoid und mindestens ein Oxim umfasst, zur Maskierung des Geruchs mindestens eines Amins, vorzugsweise mindestens eines Amins der Formel (I), vorzugsweise mindestens eines sekundären oder tertiären Amins der Formel (I), noch weiter bevorzugt eines tertiären Amins der Formel (I) nach Anspruch 2.

13. Verwendung mindestens einer Zusammensetzung nach einem der Ansprüche 1 bis 11 zur Herstellung von Polymeren oder Gießereiformen, insbesondere Kernen für Gießereiformen.

14. Gießereiform, erhalten durch Vernetzen eines mit einem Zuschlagstoff gemischten Bindemittels mit Hilfe einer Zusammensetzung nach einem der Ansprüche 1 bis 11.

## Claims

1. Composition comprising:
a) at least 90% by weight, more preferentially still at least 95%, advantageously at least 99%, more advantageously still at least 99.5% by weight of at least one primary, secondary or tertiary amine, relative to the total weight of the composition, and
b) at most 10%, more preferentially still at most 5%, advantageously at most 1%, more advantageously still at most 0.5%, particularly preferably at most 0.25%, more preferably still at most 0.2%, particularly preferably at most 0.1%, more preferably still at most 0.08% by weight of an odour-masking agent (b), relative to the total weight of the composition;
in which said odour-masking agent comprises:
- at least one ether of formula (b1):
R₄-O-R₅ (b1)
in which:
• R₄ and R₅, which may be identical or different, are selected independently of one another from a linear or branched alkyl radical comprising from 1 to 12 carbon atoms, a cycloalkyl radical comprising from 3 to 12 carbon atoms, an alkenyl radical comprising from 2 to 12 carbon atoms, a cycloalkenyl radical comprising from 3 to 12 carbon atoms, in which the alkyl, cycloalkyl, alkenyl, and cycloalkenyl radicals may optionally be substituted by one or more groups selected from hydroxyl, alkoxy, alkylcarbonyl, alkoxycarbonyl, phenyl, benzyl, fluorine, chlorine, bromine, iodine, sulphur, phosphorus and nitrogen, and preferably selected from hydroxyl, alkoxy, phenyl, benzyl, fluorine, chlorine, bromine, phosphorus and nitrogen, a phenyl radical and a benzyl radical,
• R₄ and R₅ optionally, together and with the oxygen atom to which they are bonded, forming a ring structure comprising from 3 to 20 atoms, and optionally comprising one or more heteroatoms selected from oxygen, nitrogen, sulphur and phosphorus, said ring structure being optionally substituted with one or more groups selected from hydroxyl, alkoxy, alkylcarbonyl, alkoxycarbonyl, phenyl, benzyl, fluorine, chlorine, bromine, iodine, sulphur, phosphorus and nitrogen,
- at least one terpene and/or one terpenoid (b2) and
- at least one oxime of formula (b3): 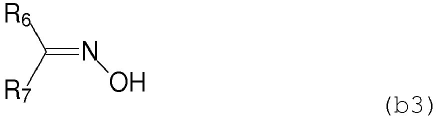 in which:
• R₆ is selected from a linear or branched alkyl radical comprising from 1 to 24 carbon atoms, a cycloalkyl radical comprising from 3 to 24 carbon atoms, an alkenyl radical comprising from 2 to 24 carbon atoms, a cycloalkenyl radical comprising from 3 to 24 carbon atoms, the phenyl radical and the benzyl radical, and R₇ is selected from a hydrogen atom, and a linear or branched alkyl radical comprising from 1 to 24 carbon atoms, a cycloalkyl radical comprising from 3 to 24 carbon atoms, an alkenyl radical comprising from 2 to 24 carbon atoms, a cycloalkenyl radical comprising from 3 to 24 carbon atoms, the phenyl radical and the benzyl radical, in which the alkyl, cycloalkyl, alkenyl, and cycloalkenyl radicals may optionally be substituted by one or more groups selected from hydroxyl, alkoxy, alkylcarbonyl, alkoxycarbonyl, phenyl, benzyl, fluorine, chlorine, bromine, iodine, sulphur, phosphorus and nitrogen, and preferably selected from hydroxyl, alkoxy, phenyl, benzyl, fluorine, chlorine, bromine, phosphorus and nitrogen,
R₆ and R₇ optionally, together and with the carbon atom to which they are bonded, forming a ring structure comprising from 3 to 20 atoms, and optionally comprising one or more heteroatoms selected from oxygen, nitrogen, sulphur and phosphorus, said ring structure being optionally substituted with one or more groups selected from hydroxyl, alkoxy, phenyl, benzyl, fluorine, chlorine, bromine, iodine, sulphur, phosphorus and nitrogen.

2. Composition according to Claim 1, in which said at least one amine is an amine of formula (I): 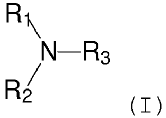 in which:
- R₁, R₂ and R₃, which may be identical or different, are selected independently of one another from a hydrogen atom, a linear or branched alkyl radical comprising from 1 to 12 carbon atoms and a cycloalkyl radical comprising from 3 to 12 carbon atoms, in which the alkyl and cycloalkyl radicals may optionally be substituted by one or more groups selected from hydroxyl, alkoxy, alkylcarbonyl, alkoxycarbonyl, phenyl, benzyl, fluorine, chlorine, bromine, iodine, sulphur, phosphorus and nitrogen, and preferably selected from hydroxyl, alkoxy, phenyl, benzyl, fluorine, chlorine, bromine, phosphorus and nitrogen,
- two of the substituents selected from R₁, R₂ and R₃ optionally, together and with the nitrogen atom to which they are bonded, forming a ring structure comprising from 2 to 12 carbon atoms, and optionally comprising one or more heteroatoms selected from oxygen, nitrogen, sulphur and phosphorus, and being optionally substituted with one or more functional groups selected from hydroxyl, alkoxy, alkylcarbonyl, alkoxycarbonyl, benzyl, fluorine, chlorine, bromine, iodine, sulphur, phosphorus and nitrogen.

3. Composition according to Claim 1, comprising at least one amine of formula (I) according to Claim 2.

4. Composition according to Claim 1, in which said at least one amine is selected from propan-1-amine, propan-2-amine, cyclopentanamine, 2-methylpropan-2-amine, phenylmethanamine, 2-aminopentane, 3-aminopentane, 1,2-dimethylpropylamine, hexylamine, 1,3-dimethylbutylamine, n-heptylamine, n-octylamine, 2-aminooctane, 3,3,5-trimethylcyclohexylamine, ethylamine (MEA), isopropylamine, *sec*-butylamine, 3-ethoxypropylamine, 3-(2-methoxyethoxy)propylamine, 3-butoxypropylamine, 3-(2-ethylhexyloxy)propylamine, 3-isopropoxypropylamine and 3-methoxypropylamine, and also mixtures of two or more thereof in any proportions.

5. Composition according to Claim 1, in which said at least one amine is selected from N-methylethanamine, N-ethylethanamine, N-methylpentan-3-amine, N-3-dimethylbutan-2-amine, di(*sec*-butyl)amine, diamylamine, isopropylbenzylamine, dihexylamine, diethylamine, diisopropylamine, N-isopropylmethylamine, N-butylmethylamine, N-(*sec*-butyl)methylamine, N-isobutylmethylamine, N-(*tert*-butyl)methylamine, N-methylpentylamine, N-hexylmethylamine, N-methylcyclohexylamine, N-heptylmethylamine, N-octylmethylamine, N-ethylmethylamine, N-ethylpropylamine, N-ethylisopropylamine, N-butylethylamine, N-(sec-butyl)ethylamine, N-ethylcyclohexylamine and N-ethylbenzylamine, and also mixtures of two or more thereof in any proportions.

6. Composition according to Claim 1, in which said at least one amine is selected from trimethylamine, N-methylaziridine, dimethylethylamine (DMEA), N-methylazetidine, N-ethylaziridine, diethylmethylamine (DEMA), dimethylisopropylamine (DMIPA), dimethyl(n-propyl)amine (DMPA), N-(n-propyl)aziridine, N-isopropylaziridine, N-ethylazetidine, N-methylpyrrolidine, N,N,N',N'-tetramethyldiaminomethane, triethylamine (TEA), methylethyl(n-propyl)amine, methylethylisopropylamine, dimethyl(n-butyl)amine, dimethyl(sec-butyl)amine, dimethylisobutylamine, dimethyl(tert-butyl)amine, N-ethylpyrrolidine, N-methylpiperidine, hexamethylenetetramine, dimethylpiperazine, N,N,N',N'-tetramethyldiaminoethane, dimethylpentylamines, methylethylbutylamines, diethylamines, dipropylmethylamines, N-propylpyrrolidines, N-ethylpiperidine, dimethylhexylamines, methylethylpentylamines, diethylbutylamines, dipropylethylamines, N-butylpyrrolidines, N-propylpiperidines, diethylpiperazine, dimethylheptylamines, methylethylhexylamines, diethylpentylamines, tripropylamines, N-pentylpyrrolidines, N-butylpiperidines, dimethyloctylamines, methylethylheptylamines, diethylhexylamines, ethylpropylpentylamines, dipropylbutylamines, N-pentylpiperidines and ethyldiisopropylamine, and also mixtures of two or more thereof in any proportions.

7. Composition according to Claim 1, in which the amine is selected from DMEA, DMIPA, DEMA, DMPA and TEA, and also mixtures of two or more thereof in any proportions.

8. Composition according to any one of the preceding claims, in which the amine is DMEA, and also mixtures of DMEA with one or more other amines of formula (I).

9. Composition according to any one of the preceding claims, in which the odour-masking agent comprises at least one ether (b1) selected from phenoxybenzene, diphenyl ether, methoxynaphthalenes, 1-methoxy-4-methyl-(4-methylanisole), 2-(2,4-dimethylcyclohex-3-en-1-yl)-5-methyl-(1-methylpropyl)-1,3-dioxane, methoxymethane, methoxyethane, ethoxyethane, 2-ethoxypropane, oxacyclopropane, oxacyclopentane, oxacyclohexane, 1,4-dioxacyclohexane, dimethoxymethane, diisopropyl ether, tetrahydrofuran, 1,4-dioxane, pyrans, dihydropyrans, phenylpyrans, dihydrophenylpyrans, and pyrans and dihydropyrans substituted by a phenyl and an alkyl and others, and also mixtures of two or more thereof in any proportions.

10. Composition according to any one of the preceding claims, in which the odour-masking agent comprises at least one terpene/terpenoid (b2) selected from terpinenes, myrcene, limonene, terpinolene, pinenes, sabinene, camphene, ocimene, eucalyptol, citral, menthol, camphor, menthone, terpineol, isoborneol, nerol, citronellal, citronellol, linalool, geraniol and myrcenol, and also mixtures of two or more thereof in any proportions, and also essential oils based on terpenes and/or terpenoids, especially those comprising these constituents.

11. Composition according to any one of the preceding claims, in which the odour-masking agent comprises at least one oxime (b3) selected from cinnamaldehyde oxime, 2-methylbutanal oxime, 3-methylbutanal oxime, methyl ethyl ketoxime, 3-heptanone oxime, 5-methyl-3-heptanone oxime, glyoxime, dimethylglyoxime, diaminoglyoxime, pralidoxime, obidoxime, perillartine, asoxime chloride and salicylaldoxime, and also mixtures of two or more thereof in any proportions.

12. Use of at least one odour-masking agent (b) comprising at least one ether, at least one terpene and/or one terpenoid and at least one oxime, to mask the odour of at least one amine, preferably at least one amine of formula (I), preferably at least one secondary or tertiary amine of formula (I), more preferably still at least one tertiary amine of formula (I) according to Claim 2.

13. Use of at least one composition according to one of Claims 1 to 11 for the manufacture of polymers or foundry moulds, in particular of cores for foundry moulds.

14. Foundry mould obtained by crosslinking a binder mixed with an aggregate by means of a composition according to any one of Claims 1 to 11.
